# EUROPÄISCHE PATENTANMELDUNG

(11) **EP 2 703 376 A1**
(43) Veröffentlichungstag der Anmeldung: **05.03.2014**
(21) Anmeldenummer: 12182669.7
(22) Anmeldetag: 31.08.2012
(51) Int. Cl.: C07C 29/20, C07C 29/56

(54) **Verfahren zur Herstellung von Menthol**

(71) Anmelder: LANXESS Deutschland GmbH, 50569 Köln (DE)
(72) Erfinder: Mechelhoff, Martin, 50733 Köln (DE); Jentsch, Jörg-Dietrich, 51381 Leverkusen (DE); Dreisbach, Claus, 42799 Leichlingen (DE); Heuer, Lutz, 41542 Dormagen (DE); Wasserscheid, Peter, 91054 Erlangen (DE); Rittsteiger, Anne, 91054 Erlangen (DE)

(57) **Zusammenfassung**

Die Erfindung betrifft ein Verfahren zur Herstellung von 2-Isopropyl-5-methylcyclohexanol (Menthol) über die Hydrierung von Thymol zu Neomenthol und der anschließende Isomerisierung zum D/L (+/-)-Menthol.

## Beschreibung

Die Erfindung betrifft ein Verfahren zur Herstellung von 2-Isopropyl-5-methylcyclohexanol (Menthol) über die Hydrierung von Thymol zu Neomenthol und der anschließenden Isomerisierung zum D/L (+/-)-Menthol (Menthol).

2-Isopropyl-5-methylcyclohexanol besitzt drei stereogene Zentren, deshalb gibt es acht Stereoisomere: D,L-Menthol, D,L-Neomenthol, D,L-Isomenthol und D,L-Neoisomenthol.

Unter den natürlich vorkommenden cyclischen Terpenalkoholen nimmt das L-Menthol, der Hauptbestandteil des Pfefferminzöls, aufgrund seiner kühlenden und erfrischenden Wirkung eine Sonderstellung ein. L-Menthol findet daher Verwendung als Riech- oder Geschmackstoff und wird in der Arzneimittelindustrie eingesetzt. Es ist deshalb das wirtschaftlich bedeutendste der Menthol-Stereoisomeren. Daher ist man generell bestrebt, die Hydrierung durch geeignete Wahl der Reaktionsbedingungen und der Katalysatoren so zu führen, dass möglichst viel D,L-Menthol entsteht.

Viele Stoffgemische, deren Komponenten nur geringe Siedepunktsunterschiede aufweisen oder gar Azeotrope bilden, sind durch konventionelle Rektifikation nur schwer bzw. gar nicht trennbar. Dies trifft auf die Trennung von Diastereomeren des 2-Isopropyl-5-methylcyclohexanols zu, wie sie typischerweise bei der Hydrierung von Thymol oder nachfolgenden Aufarbeitungsschritten entstehen. Insbesondere die Trennung der Diastereomeren Isomenthol und Menthol ist aufgrund der geringen relativen Flüchtigkeit der beiden Verbindungen zueinander nur unzureichend und unter hohem Energieeinsatz zu leisten.

Die Siedepunkte von D,L-Isomenthol (218,6°C bei 1013 hPa; 75 bis 78°C bei 3,3 hPa) und D,L-Menthol (216,5°C bei 1013 hPa; 75 bis 78°C bei 3,3 hPa) liegen sehr nahe beieinander. Die Trennleistung einer Kolonne bei der destillativen Trennung der einzelnen Menthol-Isomere wird daher insbesondere durch das Verhältnis von D,L-Menthol zu D,L-Isomenthol bestimmt. Für eine hohe Raum-Zeit-Ausbeute an D,L-Menthol bei der destillativen Trennung ist deshalb neben einem möglichst hohen D,L-Menthol-Gehalt im zu trennenden Gemisch auch ein möglichst geringer D,L-Isomenthol-Gehalt nötig. Die Ausbeute an Menthol wird also bei einer gegebenen Destillationskolonne wesentlich durch das Eingangsverhältnis von D,L-Menthol zu D,L-Isomenthol bestimmt.

Zur Herstellung von D,L-Menthol ist es bekannt, Verbindungen, die das Kohlenstoffgerüst des Menthans mit wenigstens einer Doppelbindung besitzen und in 3- Stellung durch Sauerstoff substituiert sind, wie beispielsweise Thymol, in kontinuierlichen Prozessen an festen Katalysatorschüttungen mit Wasserstoff zu hydrieren bzw. Stereoisomere des Menthols an festen Katalysatorschüttungen umzulagern.

In DE 23 14 813 A1 wird ein Verfahren zur Hydrierung von Verbindungen, die das Kohlenstoffgerüst des Menthans mit wenigstens einer Doppelbindung besitzen und in 3-Stellung durch Sauerstoff substituiert sind, an einer Schüttung eines Kobalt-Mangan-Katalysators bei Temperaturen von 170°C bis 220°C und einem Druck von über 25 bar, bevorzugt über 200 bar beschrieben. In den Beispielen wird bei Temperaturen von 180°C bis 210°C und bei Drucken über 200 bar gearbeitet und dabei ein Gemisch der acht stereoisomeren Menthole erhalten, das zu 59,5 bis 59,9 % aus dem racemischen D,L-Menthol und zu 10,6 bis 10,8 % aus D,L-Isomenthol besteht. Das Menthol-Isomenthol-Verhältnis beträgt dabei maximal 5,7. Durch Modifikation des Kobalt-Mangan-Katalysators mit Kupfer wurden Menthol-Gemische mit D,L-Menthol-Gehalten von 57,6 % und D,L- Isomenthol-Gehalten von 9,2% erreicht, was einem Menthol-Isomenthol-Verhältnis von etwa 6,3 entspricht. Die erhaltenen Gemische weisen jedoch 4 bis 5 % an unerwünschten Nebenprodukte in Form von nicht wiederverwertbaren Kohlenwasserstoffen auf.

Aus EP 0 563 611 A 1 und DE 197 18 116 A 1 ist bekannt, dass die Hydrierung von aromatischen oder teilhydrierten cyclischen Verbindungen, die das Kohlenstoffgerüst des Menthans mit wenigstens einer C=C-Doppelbindung besitzen und in 3-Stellung durch Sauerstoff substituiert sind, mit Wasserstoff an einem Festbettkatalysator durchgeführt werden kann, der Palladium, Ruthenium oder Rhodium oder ein Gemisch dieser Elemente als Aktivbestandteile und Alkalimetallhydroxide und/oder -sulfate als Promotoren jeweils aufgebracht auf einem Träger enthält, wobei der Träger mit einem Metall der Seltenen Erden und Mangan dotiert ist. In den Beispielen wurde bei Temperaturen von 180 bis 240°C und Drucken von 270 bis 300 bar gearbeitet. Dabei wurden Menthol-Gemische erhalten, die ca. 52 bis 57 % D,L-Menthol und 11,5 bis 14,8 % D,L-Isomenthol gebildet, was einem Menthol-Isomenthol-Verhältnis von 3,6 bis 4,4 entspricht.

In EP 743 296 A 1 werden Katalysatoren offenbart, die aus trägerfreien, verpressten Pulvern von Kobalt-, Mangan- und Erdalkalioxiden bzw. -hydroxiden bestehen und bei Temperaturen von 150°C bis 230°C und Drucken von 25 bis 350 bar eingesetzt werden.

Die Umlagerung von Stereoisomeren des 1-Menthols ist in US 5 756 864 beschrieben: Bei Temperaturen von 200 bis 350°C und Wasserstoff-Drucken von 50 bis 350 bar, bevorzugt 100 bis 300 bar wird D-Menthol in einem kontinuierlichen Prozess an einem Katalysator racemisiert und isomerisiert, wobei der Katalysator aus trägerfreien, gepressten Pulvern von Nickel-, Mangan- und Erdalkalihydroxiden bzw. -oxiden besteht. Dabei wurden Menthol-Gemische erhalten, die maximal zu 59,8 % aus D,L- Menthol bestanden.

Aus US 2 843 636 ist bekannt, die Isomerisierung von Stereoisomeren des Menthols zu D,L-Menthol mit Wasserstoff in Gegenwart eines Hydrierkatalysators aus der Gruppe Kupferchromit, Kobalt und Nickel bei 260 bis 280°C und 500 bis 1300 p.s.i.g. (34 bis 90 bar) in Autoklaven durchzuführen. Die entstehenden Gemische wiesen neben ca. 10 bis 12 % D,L-Isomenthol einen D,L-Menthol-Gehalt von 60 bis 64 % auf.

In DE 198 53 562 A wird eine Niederdruck-Hydrierung von Thymol an einer stationären Katalysatorschüttung beschrieben, die einen Temperaturgradienten aufweist: Die ersten zwei von fünf in Serie geschalteten Reaktorrohren werden auf 180°C temperiert, die hinteren drei Reaktorrohre auf 80 bis 90°C. Mit einem Katalysator, der auf einem Träger, der mit einem Metall der Seltenen Erden und mit Mangan dotiert ist, Ruthenium als Aktivbestandteil und Alkalimetallhydroxide als Promotoren enthält, konnte bei einem Druck von 3 bar ein Menthol-Isomerengemisch erhalten werden, das 64,4 Gew-% Menthol und 12,1 % Isomenthol enthielt, was einem Menthol-Isomenthol-Verhältnis von 5,3 entspricht. Die Isomerisierung einer wasserstoffgesättigten Mischung aus D,L-Neomenthol, D,L-Isomenthol und D,L-Menthol lieferte bei Normaldruck ein Isomerengemisch mit einer Zusammensetzung von 65,3 % D,L- Menthol und 12,1 % Isomenthol. Bei diesem Niederdruckverfahren lassen sich hohe Menthol-Gehalte von ca. 65 % erzielen. Das Menthol-Isomenthol-Verhältnis beträgt jedoch maximal 5,4.

In der DE 100 23 283 A wird nun ein verbessertes Verfahren beschrieben, in welchem Isomerengemische, die typischerweise etwa 55% D,L-Menthol aufweisen, durch Isomerisierung mit einfachen Ruthenium-Trägerkatalysatoren Menthol-reichere Gemische herstellen kann, die bis zu 67,3 % D,L-Menthol und lediglich 8,2 % D,L-Isomenthol d.h. ein Menthol-Isomenthol-Verhältnis von bis zu 8,1 aufweisen. Des Weiteren ist aus DE 100 23 283 A bekannt, dass die Katalysatoren mit Alkoholaten, Oxiden und Hydroxiden der Alkali- oder Erdalkalimetalle regeneriert werden können.

Allen bekannten Verfahren ist demnach gemeinsam, dass sie nur einen maximalen Anteil von rund 60% an D,L-Menthol ermöglichen, mindestens 8,2 % D,L-Isomethol ergeben und maximale Menthol-Isomenthol-Verhältnisse von 8,1 erlauben.

Aufgabe der Erfindung war es daher, ein selektives und technisch einfaches Verfahren für die Herstellung von D,L-Menthol in hohen Ausbeuten bereitzustellen, bei dem idealerweise keine oder nur geringe Mengen an D,L-Isomenthol entstehen und welches hohe Menthol-Isomenthol-Verhältnisse erlaubt, wobei gleichzeitig die Bildung unerwünschter Neben-produkte weitgehend vermieden wird.

Überraschenderweise konnte die Aufgabe durch eine einstufige Hydrierung erreicht werden, bei der zunächst Thymol zu einem Gemisch aus Menthol und Neomenthol hydriert 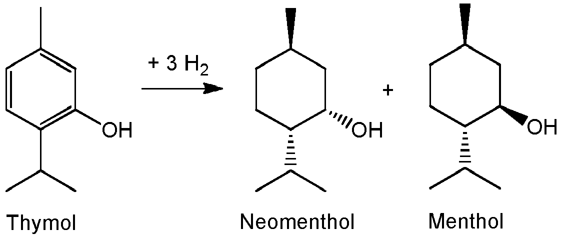 und nach destillativer Trennung das erhaltene Neomenthol mittels Katalysator zu D,L-Menthol isomerisiert wird. Gegenstand der vorliegenden Erfindung ist ein Verfahren zur Herstellung von 2-Isopropyl-5-methylcyclohexanol (Menthol), wonach
a) Thymol mit Wasserstoff in Gegenwart eines Rhodium-Katalysators, gegebenenfalls in Anwesenheit eines Lösungsmittels, hydriert wird,
b) das entstandene Neomenthol von D,L-Menthol abgtrennt und
c) das in b) isolierte Neomenthol mittels mindestens eines Ruthenium-haltigen Katalysators zu D,L-Menthol umgesetzt wird.

In einer bevorzugten Ausführungsform der vorliegenden Erfindung wird in Schritt a) auf 1 Mol Thymol ein 2 bis 150 facher Überschuss an Wasserstoff eingesetzt.

Bei den Rhodium-haltigen Katalysatoren handelt es sich vorzugsweise um metallisches Rhodium und oxidierte Spezies des Rhodium auf dem Träger Aluminiumoxid.

In einer besonders bevorzugten Ausführungsform der vorliegenden Erfindung werden die Rhodium-haltigen Katalysatoren in Teilschritt a) in Form von geträgerten oder nicht geträgerten Katalysatoren eingesetzt.

Das Trägermaterial weist vorzugsweise eine BET-Oberfläche von mindestens 100 m²/g, bevorzugt mindestens 160 m²/g, besonders bevorzugt mindestens 180 m²/g auf. Besonders bevorzugt ist Aluminiumoxid, das zusätzlich einen hohen Anteil an makroporösen Poren mit einem Porendurchmesser von mindestens 50 nm aufweist und ein Porenvolumen von mindestens 300 mm³/g, bevorzugt mindestens 600 mm³/g besitzt.

Der Anteil des Rhodium-haltigen Katalysators auf dem Trägermaterial beträgt vorzugsweise 0,3 - 10 Gew.% besonders bevorzugt 2-5 Gew.%.

Ganz besonders bevorzugt ist ein Trägermaterial aus Al₂O₃ und Silica mit einem Anteil von 2 - 5 Gew.% Rhodium.

Bei den Katalysatoren handelt es sich handelsübliche Katalysatoren, die z.B. bei der Firma Heraeus Materials Technology GmbH & Co. KG oder Johnson Matthey Plc erhältlich sind.

In einer bevorzugten Ausführungsform des erfindungsgemäßen Verfahrens wird die Hydrierung in Teilschritt a) bei Temperaturen von 60° - 250°C, vorzugsweise von 60° - 200°C, besonders bevorzugt 60 - 120°C und bei einem Druck von mindestens 1,1 bar, bevorzugt >1,1 bis 325 bar, besonders bevorzugt 2 -100 bar, durchgeführt.

In einer weiteren bevorzugten Ausführungsform der Erfindung wird die Hydrierung in Teilschritt a) in einem Lösungsmittel durchgeführt.

Als Lösungsmittel sind cyclische, verzweigte und unverzweigte Alkohole mit 1 - 10 Kohlenstoffatomen, aliphatische und cyclische Ether mit 4 - 12 Kohlenstoffatomen und/oder aliphatische und cycloaliphatische Kohlenwasserstoffe mit 5 - 12 Kohlenstoffatomen, besonders bevorzugt Methanol, Ethanol, Propanol, Isopropanol, Isobutanol, Tetrahydrofuran, Diethylenglykoldimethylether, Ethylenglykoldimethylether, Tetrahydrofuran, 1,4-Dioxan, Cyclohexan, Methylcyclohexan, Cyclooctan, Hexan, Heptan und/oder Petrolether bevorzugt.

Ganz besonders bevorzugt ist Cyclohexan.

Das Verhältnis von Thymol zu Lösungsmittel beträgt vorzugsweise 1: 0 bis 1: 20.

In einer weiteren Ausführungsform der Erfindung kann der in Schritt a) eingesetzte Katalysator rezykliert werden. Hierfür werden bevorzugt kontinuierlich durchströmte Reaktoren eingesetzt, wie z.B. Wirbelschichtreaktoren oder Reaktoren mit einem festen Katalysatorbett.

Die Abtrennung des in Schritt b) das entstandenen Neomenthols erfolgt vorzugsweise destillativ bei Temperaturen von 60 bis 150°C. Der Destillationssumpf, bestehend aus hauptsächlich Neomenthol und Menthol, wird vorzugsweise in Teilschritt a) zurückgeführt.

Die anschließende Isomerisierung des isolierten Neomenthols zu D,L-Menthol erfolgt mittels mindestens eines Ruthenium-haltigen Katalysators in Form eines geträgerten oder nicht geträgerten Katalysators.

Hierfür werden vorzugsweise die in WO2012/010695 beschriebenen Isomerisierungskatalysatoren auf Basis von Ruthenium und gegebenenfalls Erdalkalimetallalkoxylat eingesetzt, welche auf einem Trägermaterial aus Aluminiumoxid aufgebracht sind. Besonders bevorzugt ist der Einsatz von Ruthenium oder Rutheniumoxiden auf einem Trägermaterial aus Aluminiumoxid.

In einer bevorzugten Ausführungsform des erfindungsgemäßen Verfahrens wird die Isomerisierung c) bei Temperaturen von 60° - 250°C, vorzugsweise von 60° - 200°C, besonders bevorzugt 60 - 150°C und bei einem Druck von mindestens 1,1 bar, bevorzugt >1,1 bis 325 bar, besonders bevorzugt 2 - 100 bar durchgeführt.

Als Erdalkalimetallalkoxylate sind Verbindungen der Formel (I),

(R-O)₂M (I)

bevorzugt,
in denen
R jeweils unabhängig, vorzugsweise identisch für einen primären, sekundären oder tertiären, cyclischen oder acylischen, verzweigten oder unverzweigten C₁ bis C₂₀-Alkylrest steht, der gegebenenfalls durch Aryl, C₁-C₄-Alkoxyl oder C₆ bis C₁₄-Aryloxy weiter substituiert sein kann und besonders bevorzugt für Methyl, Ethyl, n-Propyl, Isopropyl, n-Butyl, sec.-Butyl, tert. Butyl, n-Pentyl, Neopentyl, n-Hexyl, Cyclohexyl oder die stereoisomeren Methylreste steht und
M für Calcium, Strontium oder Barium, vorzugsweise Barium steht.

Die bevorzugte Bariumalkoxylate können beispielsweise durch Umsetzung von Bariumperchlorat mit den entsprechenden Kaliumalkoxylaten vorzugsweise gelöst im gleichen oder einem anderen Alkohol erhalten werden, wobei sich schwerlöslichen Kaliumperchlorat bildet, welches aus den Reaktionslösungen beispielsweise durch Filtration leicht entfernt werden kann.

Bariummentholate sind beispielsweise auch erhältlich durch Versetzen von Bariumethoxid oder Bariumisopropoxid mit einem Überschuß an Menthol-Stereoisomeren und längerem Stehenlassen oder Erwärmen.

Besonders bevorzugt finden Bariumethoxid, 10% w/v in Ethanol, Bariumisopropoxid als feste Substanz, gelöst in Mentholisomeren oder Bariumisopropoxid, 20% w/v in Isopropanol Verwendung.

Das als Trägermaterial eingesetzte Aluminiumoxid kann in allen bekannten Modifikationen, bevorzugt in der γ-Modifikation eingesetzt werden. Vorteilhaft weist das als Trägermaterial eingesetzte Aluminiumoxid eine BET-Oberfläche von mindestens 100 m²/g, bevorzugt mindestens 160 m²/g, besonders bevorzugt mindestens 180 m²/g auf. Besonders bevorzugt ist Aluminiumoxid, das zusätzlich einen hohen Anteil an makroporösen Poren mit einem Porendurchmesser von mindestens 50 nm aufweist und ein Porenvolumen von mindestens 300 mm³/g, bevorzugt mindestens 600 mm³/g besitzt. Als geeignete Trägermaterialien seien beispielhaft die kommerziell erhältlichen Aluminiumoxide SPH 1515, SPH 531, SPH 501 der Firma Rhodia, D 10-10 der Firma BASF und SA 6176 der Firma Norton genannt.

Das Trägermaterial kann beispielsweise in Form von Pulver mit Korngrößen von 0,001 bis 0,1 mm, gebrochenem und gesiebtem Material mit Korngrößen zwischen 0,05 bis 5 mm oder in Formkörpern, vorzugsweise Strangpresslingen, Extrudaten, Pillen, Kugeln oder Granulaten mit Durchmessern von 0,2 bis 30 mm, eingesetzt werden.

Der besondere Vorteil des erfindungsgemäßen Verfahrens ist, dass in effizienter Weise Mischungen von Diastereomeren der 2-Isopropyl-5-methylcyclohexanolen derart getrennt werden können, dass die Diastereomeren Neomenthol und D,L-Menthol mit hohen Reinheiten erhalten werden.

In dem erfindungsgemäßen Verfahren kann der spezifische Energieverbrauch deutlich gesenkt und die Abmaße der verwendeten Trennapparate, d.h. das notwendige Apparatevolumen je erforderlicher Trennstufe, erheblich reduziert werden.

Der Rahmen der Erfindung erfasst alle oben stehenden und im Folgenden aufgeführten allgemeinen oder in Vorzugsbereichen genannten Restedefinitionen, Indizes, Parameter und Erläuterungen untereinander, also auch zwischen den jeweiligen Bereichen und Vorzugsbereichen in beliebiger Kombination.

Die nachfolgenden Beispiele dienen der Erläuterung der Erfindung, ohne dabei limitierend zu wirken.

### Beispiele:

### Beispiel 1:

6 g Thymol ( 40 mmol) wurden mit einem mindestens 3 molarem Überschuss an Wasserstoff in Gegenwart von 1 mol% Katalysator (siehe nachstehende Tabelle) bei Temperaturen von 120°C und bei einem Druck von 30 bar in Gegenwart von Cyclohexan als Lösungsmittel zu Neomenthol (Neo) hydriert.

| **Katalysatoren** | **Neomenthol-Anteil / %** | **Neo/Iso** | **Reaktionszeit t / h** |
|---|---|---|---|
| 5 % Rh/Alox, Merck KGaA (0,5 mol-%) | 49,0 | 15,5 | 1,50 |
| 5 % Rh/aktiviertes Alox, Aldrich (0,5 mol-%) | 39,7 | 11,1 | 0,25 |

Mit Adrich = Sigma-Aldrich Chemie GmbH und Neo/Iso= Neomenthon/Iosmenthol und Alox= Aluminiumoxid

Neomenthol wurde bei einer Sumpftemperatur von 142°C destillativ abgetrennt.

Die beiden Katalysatoren zeigten mit rund 40 % bzw. 49 % sehr hohe Neomenthol-Selektivitäten. Auch das Neo/Iso-Verhältnis war bei diesen Versuchen mit 11,1 (5 % Rh/aktiviertes Alox, Aldrich) bzw. 15,5 (5 % Rh/Alox, Merck KGaA) sehr hoch. Für eine technische Anwendung ist jedoch nicht nur die Selektivität und das Neo/Iso-Verhältnis, sondern auch die Reaktionsdauer entscheidend. Das Verhältnis 11,1 wurde bereits nach 25 Min, 15,5 erst nach 1,5 h erreicht.

Für die Isomerisierung wurde Neomenthol mit 1 mol% RuO₂ in Cyclohexan bei Temperaturen von 75°C bzw. 100°C und einem Druck von 10 bar umgesetzt.

| **Temperatur T / °C** | **Zeit t / h** | **Anteil Neo-Menthol / %** | **Anteil D,L-Menthol / %** |
|---|---|---|---|
| 75 °C | 22 h | 32 | 68 |
| 100 °C | 7 h | 33 | 67 |

Bei einer Reaktionstemperatur von 100°C konnte nach spätestens 7 Stunden Reaktionszeit das thermodynamische Gleichgewicht zwischen Neo- und D,L-Menthol erzielt werden. Dabei waren mit weniger als 1 % vernachlässigbare Mengen an Nebenprodukten und anderen Mentholisomeren entstanden.

Die Konzentrationsangaben beziehen sich auf GelchromatographieFlächen-%.

## Patentansprüche

1. Verfahren zur Herstellung von 2-Isopropyl-5-methylcyclohexanol (Menthol), **dadurch gekennzeichnet, dass**
a) Thymol mit Wasserstoff in Gegenwart eines Rhodium-Katalysators, gegebenenfalls in Anwesenheit eines Lösungsmittels, hydriert wird,
b) das entstandene Neomenthol von Menthol abgtrennt und
c) das in b) isolierte Neomenthol mittels mindestens eines Ruthenium-haltigen Katalysators in geträgerter oder nicht geträgerter Form zu Menthol umgesetzt wird.

2. Verfahren nach Anspruch 1, **dadurch gekennzeichnet, dass** Hydrierung in Stufe a) bei Temperaturen von 60° bis 250°C, vorzugsweise von 60° - 200°C, besonders bevorzugt 60- 120°C und bei einem Druck von mindestens 1,1 bar, bevorzugt >1,1 bis 325 bar, besonders bevorzugt 2 - 100 bar, durchgeführt wird.

3. Verfahren nach Anspruch 1 oder 2, **dadurch gekennzeichnet, dass** als Ruthenium-Katalysatoren geträgerte Katalysatoren, vorzugsweise auf Al₂O₃ geträgerte Katalysatoren, eingesetzt werden.

4. Verfahren nach einem oder mehreren der Ansprüche 1 bis 3, **dadurch gekennzeichnet, dass** als Lösungsmittel cyclische, verzweigte und unverzweigte Alkohole mit 1 - 10 Kohlenstoffatomen, aliphatische und cyclische Ether mit 4 - 12 Kohlenstoffatomen und/oder aliphatische und cycloaliphatische Kohlenwasserstoffe mit 5 - 12 Kohlenstoffatomen, vorzugsweise Methanol, Ethanol, Propanol, Isopropanol, Isobutanol, Tetrahydrofuran, Diethylenglykoldimethylether, Ethylenglykoldimethylether, Tetrahydrofuran, 1,4-Dioxan, Cyclohexan, Methylcyclohexan, Cyclooctan, Hexan, Heptan und/oder Petrolether, eingesetzt werden.

5. Verfahren nach einem oder mehreren der Ansprüche 1 bis 4, **dadurch gekennzeichnet, dass** Schritt c) bei Temperaturen von 60° - 250°C, vorzugsweise von 60° - 200°C, besonders bevorzugt 60 - 120°C, und bei einem Druck von mindestens 1,1 bar, bevorzugt >1,1 bis 325 bar, besonders bevorzugt 2 - 100 bar, durchgeführt wird.
